# EUROPEAN PATENT SPECIFICATION

(11) **EP 2 854 748 B1**
(45) Date of publication and mention of the grant of the patent: **02.08.2017**
(21) Application number: 13739807.9
(22) Date of filing: 04.06.2013
(51) Int. Cl.: A61K 8/34, A61K 8/46, A61K 31/045, A61K 31/10, A61Q 19/00, A61P 17/00

(54) **PHARMACEUTICAL COMPOSITION FOR THE TREATMENT OF SKIN INFLAMMATION AND RELATED SYNDROMES**
PHARMAZEUTISCHE ZUSAMMENSETZUNG ZUR BEHANDLUNG VON HAUTENTZÜNDUNGEN UND VERWANDTEN SYNDROMEN
COMPOSITION PHARMACEUTIQUE POUR LE TRAITEMENT DE L'INFLAMMATION CUTANÉE ET DE SYNDROMES AFFÉRENTS

(30) Priority: 04.06.2012 IT BS20120093
(43) Date of publication of application: 08.04.2015
(73) Proprietor: General Topics S.R.L., 25087 Salo (Brescia) (IT)
(72) Inventor: DE PAOLI AMBROSI, Gianfranco, I-25087 Salò (BS) (IT)
(74) Representative: Sangiacomo, Ines
(86) International application number: PCT/IB2013/054608
(87) International publication number: WO 2013/182998

(56) References cited:
- WO-A1-94/05272
- WO-A2-2005/115546
- US-A1- 2012 094 965
- KUEPER THOMAS ET AL: "Inhibition of TRPV1 for the treatment of sensitive skin.", EXPERIMENTAL DERMATOLOGY NOV 2010, vol. 19, no. 11, November 2010 (2010-11), pages 980-986, XP002692505, ISSN: 1600-0625

## Description

### Field of the invention

The present invention relates to a composition for cosmetic or pharmaceutical use; particularly, the present invention relates to a composition for pharmaceutical use which is intended for an external use and to be applied to either an intact or injured skin or to mucous membranes for the treatment of rosacea and, more generally, of inflammation-based skin syndromes, and for the treatment of the erythema induced by ultraviolet radiations and, more generally, by the effect of ultraviolet radiations on a skin already affected by an inflammatory process.

### State of the art

There are known various inflammatory conditions of the skin which lead to an over-expression of cytokines, angiokines and inflammatory chemical mediators in general, and which involve a specific activation of specific membrane receptors which can alter ion exchanges.

Rosacea, one of these inflammatory conditions, is a chronic pathology mainly affecting the face, including cheeks, chin, nose and forehead, and it is often characterized by remission and exacerbation and comprises a series of symptoms such as intermittent erythema, permanent erythema, formation of telangiectasias, edema, papules, pustules and ocular lesions.

Rosacea affects individuals of both male and female gender, and it usually appears at an age of more than 30 years.

The exact nosology of rosacea has not been yet established. The Scientific Committee of the US "National Rosacea Society" defined 4 sub-types of Rosacea.

### Sub-type 1 - erythematotelangiectatic rosacea:

It is primarily characterized by intermittent or permanent erythema in the central portion of the face. The presence of telangiectasias, i.e. dilations of small blood vessels, is common but not essential for the diagnosis of this sub-type. Edema, desquamation, roughness and burning and itching sensations can also be observed. A history of intermittent erythema is common for individuals with erythematotelangiectatic rosacea.

### Sub-type 2 - papulopustular rosacea:

This sub-type is characterized by the presence of persistent erythema with papules and pustules which are transitory in nature and generally, but not exclusively, restricted to the central portion of the face. In many aspects, it may be confused with acne vulgaris, except only for the lack of comedones, also known as blackheads. Rosacea and acne may occur simultaneously. Sub-type 2 is often associated with sub-type 1.

### Sub-type 3 - phymatous rosacea:

This sub-type of rosacea includes symptoms such as thickened skin, uneven skin surface with the presence of nodular elements and enlargement of the nose (rhinophyma), although phymatous rosacea may occur in different areas such as cheeks, forehead, chin and ears.

Sub-type 3 also often appears in association with sub-types 1 and 2, with the presence of persistent erythema and telangiectasias.

### Sub-type 4 - ocular rosacea:

A subject should be diagnosed with ocular rosacea when he/she shows any one of the following symptoms: interpalpebral conjunctival hyperemia, burning, itching, dryness, light sensitivity, telangiectasias of the conjunctiva and eyelid margins, and periocular erythema. An individual is often diagnosed with ocular rosacea when there are symptoms of rosacea.

An early treatment of rosacea is essential to be able to hinder its progression. When rosacea is left not treated, it very often worsens and the chances of therapeutic success decrease.

The Applicant has noted that, despite the availability of various therapeutic means to control inflammatory lesions such as papules and pustules, no means exist to control erythema which is very likely to represent the initial phase of the syndrome, as seen above.

The Applicant has also noted that another aspect that has to be considered as an aggravating factor is that one induced by ultraviolet radiations in determining the intensity of erythema and its transition from intermittent to permanent erythema; in fact, rosacea can be regarded as a photo-induced or at least photo-aggravated dermatitis.

The "Transient Receptor Potential cation channels of subfamily V number 1" (TrpV1), also known as the capsaicin vanilloid receptors-1, are proteins encoded by gene TRPV1 in humans.

During the progression of an inflammatory process, TRPV1 receptors are activated by a variety of inflammatory chemical mediators such as cytokines, growth factors, prostaglandins, bradykinins, etc.

Among these, TRPV1, TRPV2, TRPV3, TRPV4 are found to be activated in rosacea; particularly, in the early stage of erythematotelangiectatic rosacea, features are found which are typical of a neurogenic-type inflammation characterized by the activation of TRPV1 receptors.

WO 2005/115546 discloses a cosmetic, pharmaceutical, or medical composition based on dimethyl sulfone to be applied on the skin, both integral and damaged, for the treatment of rosacea, acne, psoriasis, atopic dermatitis, seborrheic dermatitis and erythema, intending by erythema also cutaneous flushing as it is called in the cosmetics field; and the use of dimethyl sulfone in combination with substances having anti-bacterial and/or anti-biotic activity, and metronidazole, flavonoids, vitamins, cortisone agents, tacrolimus for said treatment.

Non patent document Kueper Thomas et Al: "Inhibition of TRPV1 for the treatment of sensitive skin.", Experimental Dermatology Nov 2010, vol. 19, no 11, November 2010 (2010-11), pages 980-986, XP002692505, ISSN: 1600-0625, discloses the TRPV1 inhibition activity of trans-4-tert-butylcyclohexanol in the treatment of sensitive skin, and it discloses that the cis-4-tert-butylcyclohexanol does not show TRPV antagonist activity.

### Summary of the invention

Therefore, the aim of the present invention is to counter inflammatory processes of the skin in which an increased production of chemical inflammatory mediators and an up-regulation of TRPV membrane receptors occur simultaneously.

Particularly, the aim of the present invention is to act to simultaneously hinder both the release of chemical mediators which are up-regulated in rosacea and their action of causing the activation of TRPV receptors.

Therefore, in a first aspect, the present invention relates to a pharmaceutical composition such as that set forth in claim 1.

Particularly, the pharmaceutical composition of the present invention comprises a mixture consisting of methyl sulfonyl methane (also known as dimethyl sulfone) and at least one compound belonging to the class of 4-alkyl cyclohexanols, and it is useful in the treatment of various inflammatory conditions such as rosacea.

Indeed, the composition of the present invention is used on one hand for its ability of inhibiting the release of inflammatory chemical mediators, thereby hindering the activation of TRPV receptors, and on the other hand for its ability of hindering the activation of TRPV receptors themselves, thereby significantly decreasing the intensity of multiple discomfort sensations such as, for example, burning and itching.

Furthermore, the composition of the present invention is used for its ability of reducing the effect of ultraviolet radiations on a skin already affected by an inflammatory process and, particularly, for its ability of reducing the erythema induced by ultraviolet radiations on a skin already affected by an inflammatory process.

Preferably, said class of 4-alkyl cyclohexanols comprises 4-lower-alkyl cyclohexanols, wherein 4-lower-alkyl cyclohexanols are intended to mean 4-cyclohexanols substituted with alkyl groups having from 1 to 10 carbon atoms, more preferably from 1 to 5 carbon atoms, such as methyl, ethyl, t-butyl, propyl.

Therefore, preferably, said pharmaceutical composition of the present invention comprises a mixture consisting of methyl sulfonyl methane and at least one compound belonging to the class of 4-lower-alkyl cyclohexanols.

More preferably, said pharmaceutical composition of the present invention comprises a mixture consisting of methyl sulfonyl methane and 4-t-butyl-cyclohexanol.

Surprisingly, this composition has shown to have a particular excellent ability to act as an antagonist of TRPV epidermal receptors which are active in the erythematotelangiectatic syndrome of rosacea. Indeed, this combination is useful in achieving the double advantage of both an overall improvement of skin conditions (clear signs such as erythema, for example) and a decrease in sensations such as burning, itching, tingling, etc.

Furthermore, this combination of methyl sulfonyl methane and 4-t-butyl-cyclohexanol has also surprisingly proved to provide excellent results in treating inflammatory processes of the skin which are characterized by the presence of specific lesions such as, for example, rosacea at the papulopustular stage, in which specifically inflammatory lesions such as papules and pustules are also present. These results are attributable to the inhibition of TRPV receptors.

Preferably, the composition of the present invention comprises a suitable carrier for topical use.

Preferably, the composition of the present invention comprises methyl sulfonyl methane at a concentration in the range between 0.05% and 90% by weight, more preferably between 0.5% and 15%, even more preferably between 2% and 7.5%, based on the total weight of the mixture.

Preferably, the composition of the present invention comprises a compound belonging to the class of 4-lower-alkyl cyclohexanols at a concentration in the range between 0.05% and 90% by weight, more preferably between 0.1% and 10.0%, even more preferably between 0.5 and 5%, based on the total weight of the mixture.

Preferably, the composition of the present invention further comprises metronidazole. Preferably, said metronidazole is present at a concentration in the range between 0.05% and 1% by weight, more preferably between 0.20% and 0.75%, even more preferably between 0.4 and 0.5% by weight, based on the total weight of the mixture.

In this way, the composition of the present invention is particularly advantageous when the therapeutic action is directed to control an inflammatory condition of the skin which is characterized by erythema and actual lesions such papules and pustules.

Preferably, the composition of the present invention also comprises other ingredients which are typically found in pharmaceutical compositions such as, for example, surfactants, emulsifiers, emollients, preservatives, solvents.

Preferably, the composition according to the present invention is in the form of a cream, an unguent, an ointment, a gel, a milk, a lotion, or other typical forms used in pharmacology.

More preferably, the composition according to the present invention is in the form of a soft, non-greasy cream which is suitable for all types of skin.

In a second aspect, the present invention relates to the above described composition for use as a medicament as set forth in claim 8.

In fact, the Applicant of the present application has surprisingly found that a pharmaceutical composition as that described above can be used as a medicament for simultaneously hindering both the release of chemical mediators which are up-regulated in rosacea and their action of causing the activation of TRPV receptors.

In a third aspect, the present invention relates to the above described composition for an use as that set forth in claim 9.

In fact, the Applicant of the present application has surprisingly found that a pharmaceutical composition as that described above can be used to reduce the effect of ultraviolet radiations on a skin already affected by an inflammatory process.

In a fourth aspect, the present invention relates to the above described composition for an use as that set forth in claim 10.

In fact, the Applicant of the present application has surprisingly found that a pharmaceutical composition as that described above can be used to reduce an erythema induced by ultraviolet radiations on a skin already affected by an inflammatory process.

Said composition has to be applied to the skin by spreading it evenly and repeating the operation even several times per day.

Further characteristics and advantages of the present invention will become more apparent upon consideration of the following detailed description of a preferred but not exclusive embodiment, which is shown for illustration and not limitative purposes.

### Detailed description of the invention

The following detailed description refers to a particular embodiment of a composition according to the present invention.

### Activity of methyl sulfonyl methane.

A comparison was made between the inhibition of TNF alpha release from human keratinocytes as induced by contact with sodium lauryl sulphate (SLS) and obtained with the use of methyl sulfonyl methane (MSM), and that obtained with the use of hydrocortisone, a compound known to be active in inhibiting the release of TNF alpha. Such a comparison showed that, after a 24 hour-period under identical treatment conditions, the percentage decrease of TNF alpha release as obtained with the use of MSM was 12.33%, whereas that obtained with the use of hydrocortisone was only 5.25%. Therefore, the studies performed pointed out that MSM can decrease both the release of TNF alpha (this cytokine is expressed by keratinocytes in an inflammatory process of the skin) and the overall intensity of erythema even when TNF alpha is over-expressed by ultraviolet radiations. This result shows that methyl sulfonyl methane can result in an under-expression of such a protein even in an acute inflammation (exposure to SLS and immediate assessment), with a high potential of methyl sulfonyl methane in controlling the inflammatory process of the skin. Moreover, it should be noted that TNF alpha can activate TRPV receptors and, therefore, a decrease in the production thereof can prevent it from exerting an agonist effect on the receptors themselves.

Activity of 4-alkyl cyclohexanols. This class of molecules can act as an antagonist of TRPV receptor and, therefore, it can exert a multiple improvement effect during various inflammatory processes.

With regard to the skin, TRPV receptors have been localized at keratinocytes and sebocytes, and their activation triggers biochemical processes which lead to the perception of unpleasant sensations, such as burning and itching.

4-alkyl cyclohexanols are antagonists of TRPV receptors and, consequently, they represent a useful tool to counteract sensations occurring in an inflammatory process.

Therefore, the composition based on methyl sulfonyl methane and derivatives of 4-alkyl cyclohexanol is innovative in controlling the inflammatory process and the sensations resulting therefrom.

This composition has shown to be active in controlling the clinical picture of an inflammatory process, such as rosacea, in which the apparent damage is exacerbated by sensations which can even include pain.

### Composition examples of the invention

Composition 1. Pharmaceutical emulsion for topical use based on methyl sulfonyl methane and 4-t-butyl-cyclohexanol, comprising:

| | Weight % |
|---|---|
| - steareth 2 | 2.00 |
| - steareth 21 | 3.00 |
| - Ppg-15 stearyl ether | 10.00 |
| - stearic acid | 5.00 |
| - butylhydroxytoluene (BHT) | 0.01 |
| - 4-t-butyl-cyclohexanol | 2.50 |
| - methyl sulfonyl methane | 5.00 |
| - ethyl alcohol | 5.00 |
| - preservatives | q.s. |
| - water | q.s. |

A mixture (said phase A) comprising 2% by weight of Steareth 2, 3% by weight of Steareth 21, 10% by weight of Ppg-15 stearyl ether, 5% by weight of stearic acid, 0.01% by weight of (BHT) and 2.50% by weight of 4-t-butyl-cyclohexanol was heated to 80°C. This phase A was added with appropriate amounts of water and preservatives heated to 75°C; finally, the resulting mixture was added with a phase B consisting of 5% by weight of methyl sulfonyl methane and 5% of ethyl alcohol heated to 40°C, to obtain composition 1.

Composition 2. Emulsion for topical use based on dimethyl sulfone, 4-t-butyl-cyclohexanol and metronidazole:

| | Weight % | |
|---|---|---|
| - steareth 2 | 2.00 | emulsifier |
| - steareth 21 | 3.00 | surfactant |
| - Ppg-15 stearyl ether | 10.00 | emollient |
| - stearic acid | 5.00 | emulsifier |
| - butylhydroxytoluene (BHT) | 0.01 | preservative |
| - 4-t-butyl-cyclohexanol | 4.50 | |
| - metronidazole | 0.25 | |
| - methyl sulfonyl methane | 5.00 | |
| - ethyl alcohol | 5.00 | solvent |
| - preservatives | q.s. | |
| - water | q.s. | |

Composition 2 was prepared in a manner similar to composition 1, except that phase B also comprised 0.25% by weight of metronidazole.

### Clinical assessments

Then, some clinical assessments were carried out to demonstrate the ability of the combination of methyl sulfonyl methane and 4-t-butyl-cyclohexanol in controlling erythema and enhancing skin hydration. In this regard, it should be said that an inhibition of the activation of TRPV receptors causes an increase in differentiation capability of epidermis and a substantial improvement of its barrier functions which eventually appears as an increase in surface hydration.

First assessment. A first clinical assessment was carried out by repeatedly and continuously applying the above-described composition 1 of the invention to 12 individuals of both genders affected by rosacea, over a period of 30 days. Considering that assessments of this type, aimed to demonstrate the efficacy of a product against rosacea, require at least 90 days of therapy, this 30 day-period is quite short.

Table 1 hereinbelow shows the skin hydration values, expressed as arbitrary units of a corneometer, from 12 subjects without and with treatment with the composition 1 of the invention.

**Table 1**

| Individual | skin hydration | |
|---|---|---|
| | untreated | treated with composition 1 |
| 1 | 32 | 34 |
| 2 | 36 | 42 |
| 3 | 41 | 43 |
| 4 | 42 | 44 |
| 5 | 27 | 32 |
| 6 | 28 | 36 |
| 7 | 34 | 34 |
| 8 | 56 | 59 |
| 9 | 57 | 64 |
| 10 | 55 | 58 |
| 11 | 55 | 64 |
| 12 | 46 | 56 |
| Average 1-12 | 42 | 47 |

From Table 1 above, it is clear that patients treated with the composition 1 of the present invention, comprising the combination of methyl sulfonyl methane and 4-t-butyl-cyclohexanol, show a significant increase in skin hydration.

Second assessment. Then, a second clinical assessment was carried out, again on 12 subjects by reflectance spectrophotometry, to demonstrate the effectiveness of the composition 1 of the present invention in decreasing erythema in a patient affected by rosacea.

Table 2 hereinbelow shows the erythema values, expressed as arbitrary units, from 12 subjects without and with treatment with the composition 1 of the invention.

**Table 2**

| Individual | erythema not treated | erythema treated with composition 1 |
|---|---|---|
| 1 | 356 | 317 |
| 2 | 410 | 394 |
| 3 | 491 | 500 |
| 4 | 468 | 463 |
| 5 | 405 | 327 |
| 6 | 460 | 447 |
| 7 | 462 | 455 |
| 8 | 505 | 355 |
| 9 | 590 | 567 |
| 10 | 591 | 526 |
| 11 | 464 | 456 |
| 12 | 411 | 355 |
| Average 1-12 | 468 | 430 |

The clinical results listed in Table 2 are to be considered as a surprising consequence of the synergistic action mechanism induced by the two compounds, i.e. methyl sulfonyl methane and 4-t-butyl-cyclohexanol, in the treatment of rosacea.

Therefore, the combination of methyl sulfonyl methane and 4-t-butyl-cyclohexanol is to be considered as extremely useful in controlling the overall picture of various inflammatory processes, such as rosacea, which can occur at the skin and which are characterized by erythema and unpleasant sensations such as itching and burning.

Accordingly, the mechanism by which the combination shows its action is a synchronized, double mechanism: on one hand, due to the action of methyl sulfonyl methane, it inhibits the release of inflammatory chemical mediators (TNF alpha), thereby hindering the activation of TRPV receptors; and, on the other hand, due to the antagonist action of 4-t-butyl-cyclohexanol, it leads to a significantly reduced intensity of sensations such as itching, burning and even pain, which are common features of the general clinical picture of an inflammatory process.

In another assessment, we examined the activity against an erythema induced by ultraviolet radiations at a dose of twice the MED (Minimal Erythemogenic Dose). This test was carried out on healthy subjects, and it was evaluated by reflectance spectrophotometry. When the composition 1 of the present invention was used for the treatment, the reduction of erythema induced by ultraviolet radiations was higher than 60%.

Third assessment. Then, a third clinical assessment was carried out, by using the same procedure as followed in the second clinical assessment described above, to demonstrate the effectiveness of the composition 2 of the present invention in decreasing erythema in a patient affected by rosacea at the papulopustular stage.

This assessment is still on-going, but preliminary results have already demonstrated that, after a few days of treatment, patients administered with the composition 2 of the present invention, comprising a combination of methyl sulfonyl methane, 4-t-butyl-cyclohexanol and metronidazole, showed significant improvements in inflammation, primarily in terms of erythema, in specifically inflammatory lesions such as papules and pustules, and in subjective signs of discomfort such as tingling, burning and itching.

Many modifications and variations of the preferred embodiments will be certainly evident to those skilled in the art, without departing from the scope of the invention. Therefore, the present invention is not limited to the described preferred embodiment, shown for purposes of illustrations only and without limitation, but it is defined by the following claims.

## Claims

1. Pharmaceutical composition comprising a mixture consisting of methyl sulfonyl methane and at least one compound belonging to the class of 4-alkyl-cyclohexanols, wherein said at least one compound belonging to the class of 4-alkyl-cyclohexanols is 4-t-butyl-cyclohexanol.

2. Composition according to claim 1, which comprises methyl sulfonyl methane at a concentration of between 0.05% and 90%, and 4-t-butyl-cyclohexanol at a concentration of between 0.05% and 90% by weight, based on the total weight of the mixture.

3. Composition according to any one of claims 1-2, which comprises methyl sulfonyl methane at a concentration of between 0.5% and 15% based on the total weight of the mixture.

4. Composition according to any one of claims 1-3, which comprises 4-t-butyl-cyclohexanol at a concentration of between 0.1% and 10.0% based on the total weight of the mixture.

5. Composition according to any one of claims 1-4, further comprising metronidazole.

6. Pharmaceutical composition comprising a mixture consisting of methyl sulfonyl methane and at least one compound belonging to the class of 4-alkyl-cyclohexanols, wherein said at least one compound belonging to the class of 4-alkyl-cyclohexanols is 4-t-butyl-cyclohexanol, for use as a medicament in the treatment of an inflammatory condition of the skin **characterized by** an up-release of chemical mediators of inflammation due to rosacea and by the simultaneous up-regulation of TRPV membrane receptors.

7. Pharmaceutical composition comprising a mixture consisting of methyl sulfonyl methane and at least one compound belonging to the class of 4-alkyl-cyclohexanols, wherein said at least one compound belonging to the class of 4-alkyl-cyclohexanols is 4-t-butyl-cyclohexanol, for use as a medicament in the treatment of skin affected by an inflammatory process, to reduce the effect of ultraviolet radiations.

8. Pharmaceutical composition comprising a mixture consisting of methyl sulfonyl methane and at least one compound belonging to the class of 4-alkyl-cyclohexanols, wherein said at least one compound belonging to the class of 4-alkyl-cyclohexanols is 4-t-butyl-cyclohexanol, for use as a medicament in the treatment of skin affected by an inflammatory process, to reduce an erythema induced by ultraviolet radiations.

## Patentansprüche

1. Pharmazeutische Zusammensetzung umfassend eine Mischung aus Methylsulfonylmethan und mindestens einer Verbindung, die zur Klasse der 4-Alkyl-Cyclohexanole gehört, wobei, die mindestens eine Verbindung, die zur Klasse der 4-Alkyl-Cyclohexanole gehört, 4-t-Butyl-Cyclohexanol ist.

2. Zusammensetzung nach Anspruch 1, welche Methylsulfonylmethan in einer Konzentration zwischen 0,05% und 90% und 4-t-Butyl-Cyclohexanol in einer Konzentration zwischen 0,05 Gew.-% und 90 Gew.-% bezogen auf das Gesamtgewicht der Mischung umfasst.

3. Zusammensetzung nach einem der Ansprüche 1-2, welche Methylsulfonylmethan in einer Konzentration zwischen 0,5% und 15% bezogen auf das Gesamtgewicht der Mischung umfasst.

4. Zusammensetzung nach einem der Ansprüche 1-3, welche 4-t-Butyl-Cyclohexanol in einer Konzentration zwischen 0,1% und 10,0% bezogen auf das Gesamtgewicht der Mischung umfasst.

5. Zusammensetzung nach einem der Ansprüche 1-4, die weiter Metronidazol umfasst.

6. Pharmazeutische Zusammensetzung umfassend eine Mischung aus Methylsulfonylmethan und mindestens einer Verbindung, die zur Klasse der 4-Alkyl-Cyclohexanole gehört, wobei die mindestens eine Verbindung, die zur Klasse der 4-Alkyl-Cyclohexanole gehört, 4-t-Butyl-Cyclohexanol ist, zur Verwendung als Arzneimittel bei der Behandlung eines Entzündungszustandes der Haut, **gekennzeichnet durch** eine Hoch-Freisetzung von chemischen Mediatoren der Entzündung aufgrund von Rosacea und durch die gleichzeitige Hochregulierung von TRPV Membranrezeptoren.

7. Pharmazeutische Zusammensetzung umfassend eine Mischung aus Methylsulfonylmethan und mindestens einer Verbindung, die zur Klasse der 4-Alkyl-Cyclohexanole gehört, wobei die mindestens eine Verbindung, die zur Klasse der 4-Alkyl-Cyclohexanole gehört, 4-t-Butyl-Cyclohexanol ist, zur Verwendung als Arzneimittel bei der Behandlung der von einem Entzündungsprozess betroffenen Haut, zur Reduzierung der Wirkung von UV-Strahlungen.

8. Pharmazeutische Zusammensetzung umfassend eine Mischung aus Methylsulfonylmethan und mindestens einer Verbindung, die zur Klasse der 4-Alkyl-Cyclohexanole gehört, wobei die mindestens eine Verbindung, die zur Klasse der 4-Alkyl-Cyclohexanole gehört, 4-t-Butyl-Cyclohexanol ist, zur Verwendung als Arzneimittel bei der Behandlung der von einem Entzündungsprozess betroffenen Haut, zur Reduzierung eines durch die UV-Strahlung verursachten Erythems.

## Revendications

1. Composition pharmaceutique comprenant un mélange qui consiste en méthyl-sulfonyl-méthane et au moins un composé qui est membre de la classe des 4-alkyl-cyclohexanols, dans la quelle au moins un composé qui est membre de la classe de 4-alkyl-cyclohexanols est 4-t-butyl-cyclohexanol.

2. Composition selon la revendication 1, qui comprend méthyl-sulfonyl-méthane à une concentration entre 0,05% et 90%, et 4-t-butyl-cyclohexanol à une concentration entre 0,05% e 90% en poids, par rapport au poids total du mélange.

3. Composition selon l'une quelconque des revendications 1-2, qui comprend méthyl-sulfonyl-méthane à une concentration entre 0,5% et 15% par rapport au poids total du mélange.

4. Composition selon l'une quelconque des revendications 1-3, qui comprend 4-t-butyl-cyclohexanol à une concentration entre 0,1% et 10,0% par rapport au poids total du mélange.

5. Composé selon une l'une quelconque des revendications 1-4 comprenant en outre métronidazole.

6. Composition pharmaceutique comprenant un mélange qui consiste en méthyl-sulfonyl-méthane et au moins un composé qui est membre de la classe des 4-alkyl-cyclohexanols, dans la quelle au moins un composé qui est membre de la classe des 4-alkyl-cyclohexanols est 4-t-butyl-cyclohexanol, pour l'utilisation comme médicament dans le traitement d'une condition inflammatoire de la peau, **caractérisée par** une libération augmentée des médiateur chimiques de l'inflammation causée en raison de la rosacée e par la régulation positive simultané des récepteurs TRPV de membrane.

7. Composition pharmaceutique comprenant un mélange qui consiste en méthyl-sulfonyl-méthane et au moins un composé qui est membre de la classe des 4-alkyl-cyclohexanols, dans la quelle au moins un composé qui est membre de la classe des 4-alkyl-cyclohexanols est 4-t-butyl-cyclohexanol, pour l'utilisation comme médicament dans le traitement de la peau affectée par un processus inflammatoire, au fin de réduire l'effet des radiations ultraviolettes.

8. Composition pharmaceutique comprenant un mélange qui consiste en mëthyl-sulfonyl-méthane et au moins un composé qui est membre de la classe des 4-alkyl-cyclohexanols, dans la quelle au moins un composé qui est membre de la classe des 4-alkyl-cyclohexanols est 4-t-butyl-cyclohexanol, pour l'utilisation comme médicament dans le traitement de la peau affectée par un processus inflammatoire, au fin de réduire un érythème causé par les radiations ultraviolettes.
